# FASCICULE DE BREVET EUROPEEN

(11) **EP 3 031 429 B1**
(45) Date de publication et mention de la délivrance du brevet: **15.02.2023**
(21) Numéro de dépôt: 16154375.6
(22) Date de dépôt: 24.09.2012
(51) Int. Cl.: A61F 9/00, B05B 13/00, B65D 47/18

(54) **DISPOSITIF DE DISTRIBUTION DE LIQUIDE SOUS FORME DE GOUTTES**
VORRICHTUNG ZUR AUSGABE EINER FLÜSSIGKEIT IN TROPFENFORM
DEVICE FOR DISTRIBUTING LIQUID IN THE FORM OF DROPS

(30) Priorité: 22.09.2011 FR 1158469
(43) Date de publication de la demande: 15.06.2016
(62) Demande divisionnaire de: 12787756.1
(73) Titulaire: NEMERA LA VERPILLIERE, 38290 La Verpillière (FR)
(72) Inventeur: JULIA, Xavier, 28300 Champhol (FR); PAINCHAUD, Gaëtan, 69340 Francheville (FR); GREVIN, Guillaume, 38080 L'Isle d'Abeau (FR)
(74) Mandataire: LLR

(56) Documents cités:
- DE-B3-102009 048 476
- DE-U1- 8 910 409
- JP-U- 3 152 970
- US-A- 4 550 866
- US-A- 4 629 456

## Description

La présente invention concerne la distribution de liquide sous forme de gouttes dans le domaine pharmaceutique, par exemple du liquide ophtalmique, nasal, buccal ou auriculaire.

On entend par « produit liquide » un produit non solide et non gazeux, plus ou moins visqueux.

On connaît déjà des dispositifs de distribution comprenant un orifice de distribution d'où sortent les gouttes, ménagé par exemple au centre et à l'extrémité d'une buse de distribution, généralement de couleur blanche.

Le document US4629456 divulgue un embout cylindrique ayant une zone de démarcation colorée.

Il se trouve que la distribution de gouttes requiert une certaine précision de la part de l'utilisateur, tout particulièrement dans le cas de la distribution d'un liquide dans l'œil. Ainsi, une manipulation maladroite de l'utilisateur peut facilement conduire au dépôt de la goutte à côté de la zone visée.

La présente invention vise à proposer un dispositif de distribution permettant à l'utilisateur de distribuer les gouttes avec plus de précision.

A cet effet, l'invention a pour objet un dispositif de distribution de liquide sous forme de gouttes comprenant un orifice de distribution des gouttes ménagé sur une extrémité de distribution, dans lequel le dispositif comprend des moyens de localisation visuelle de l'orifice, par réalisation d'une zone de démarcation ayant une couleur très contrastée par rapport à d'autres parties de l'extrémité de distribution, le dispositif comprenant un embout de distribution de forme générale sensiblement cylindrique, l'extrémité de distribution ayant une forme générale de disque au centre duquel se trouve l'orifice de distribution, l'extrémité du dispositif se présentant sous forme d'une surface relativement étendue par rapport à la surface de l'orifice de distribution, l'aire de la surface de l'extrémité de distribution étant au moins deux fois supérieure à la surface de l'orifice de distribution, l'embout de distribution ayant une dimension relativement réduite dans la direction longitudinale du dispositif, le disque étant de couleur opaque.

Grâce à la zone de démarcation, l'utilisateur détecte très facilement où se trouve l'orifice de distribution, ce qui lui permet de mieux viser lors de la délivrance de la goutte. Cette zone de démarcation se révèle particulièrement intéressante pour la distribution de liquide dans l'œil (liquide ophtalmique) du fait qu'au moment de la délivrance de la goutte, le dispositif se trouve extrêmement proche de l'œil qui de ce fait, ne peut pas focaliser ou accommoder et voit le dispositif de façon très floue, sans distinguer nettement le relief du dispositif, donc l'endroit d'où la goutte va sortir. En outre dans le cas de la distribution de plusieurs gouttes consécutives dans l'œil, l'utilisateur voit encore plus flou après la première goutte reçue.

Ainsi, au lieu de réaliser un dispositif dans lequel toutes les parties ont une couleur similaire, généralement blanche, sans contraste entre elles, les inventeurs ont eu l'idée de réaliser un fort contraste visuel pour aider l'utilisateur à localiser l'orifice, même lorsqu'il voit flou. Des tests auprès d'utilisateurs ont permis de vérifier que la précision de la distribution est nettement améliorée.

On notera que l'on entend par « orifice de distribution » la partie de l'extrémité d'où sortent les gouttes, de préférence une partie de forme conique permettant de former les gouttes, disposée au centre et en partie supérieure de l'extrémité de distribution. Par ailleurs on comprend que la zone de démarcation ayant une couleur très contrastée par rapport à d'autres parties de l'extrémité de distribution peut signifier par exemple que la zone de démarcation est très foncée alors que le reste de l'extrémité de distribution est, au moins en partie, très clair ou bien que la zone de démarcation est très claire alors que le reste de l'extrémité de distribution est, au moins en partie, très foncé.

Ce mode de réalisation est particulièrement intéressant du fait que, comme l'extrémité du dispositif se présente sous forme d'une surface sensiblement plane et relativement étendue par rapport à la surface de l'orifice de distribution, à la différence d'une buse conique protubérante, il est plus difficile pour l'utilisateur de distinguer l'orifice de distribution formé au centre de cette surface, tout particulièrement si les gouttes sont distribuées au voisinage de l'œil.En outre, un tel embout sensiblement cylindrique a une dimension relativement réduite dans la direction longitudinale du dispositif, si bien que le dispositif est plus près de l'œil de l'utilisateur, d'où une plus grande difficulté pour focaliser et pour voir l'embout en trois dimensions (du fait que l'embout présente moins d'ombres), donc pour voir l'embout de façon nette. L'utilisation des moyens de localisation peut donc créer un sentiment de sécurité chez l'utilisateur.

Le disque est de couleur opaque. En d'autres termes, il est de couleur non transparente, par exemple de couleur blanche ou d'une autre couleur, ou encore présentant des motifs. Dans ce cas-là, le dispositif proposé ci-dessus est particulièrement intéressant car, lorsque le haut du dispositif est opaque et qu'il présente un disque étendu à son extrémité proche de l'oeil, il constitue une gêne supplémentaire pour l'utilisateur, car la focalisation est plus difficile que dans le cas où le disque serait transparent.

Le dispositif selon l'invention peut en outre comporter l'une ou plusieurs des caractéristiques suivantes, prises seules ou en combinaison.
- La zone de démarcation est réalisée à l'intérieur de l'orifice de distribution. En particulier, la zone de démarcation est une zone foncée disposée dans ou à l'intérieur d'une cavité de formation des gouttes, alors que l'entourage immédiat extérieur de cette cavité de formation des gouttes est réalisé dans une couleur claire.
- La zone de démarcation est disposée autour de l'orifice de distribution. En particulier, la zone de démarcation est une zone claire disposée dans ou à l'intérieur de l'orifice de distribution, délimité de préférence par une cavité conique de formation de gouttes, alors que l'entourage immédiat de l'orifice de distribution est réalisé dans une couleur foncée. Par exemple, la zone de démarcation est réalisée sous forme d'un anneau coloré, continu ou discontinu, disposé autour de l'orifice de distribution. L'anneau coloré peut être discontinu, sous forme d'une ligne circulaire hachée, par exemple par un alignement circulaire de carrés, de triangles, de ronds ou de tout autre motif.
- Le dispositif comprend des moyens de formation de gouttes et la zone de démarcation est obtenue par coloration de ces moyens de formation de gouttes, de préférence dans une couleur foncée. Ces moyens de formation de gouttes sont de préférence composés par une forme évasée conique. Selon un mode de réalisation intéressant, les moyens de formation de gouttes sont réalisés dans une partie formant valve de passage du liquide, en matériau élastomère. De façon alternative, la zone de démarcation est disposée autour des moyens de formation de gouttes, par exemple par une coloration foncée de tout ou partie d'un capot entourant les moyens de formation de goutte.
- L'embout comprend une valve de distribution de gouttes, un support de valve rapporté sur un réservoir et un capot de plaquage de la valve contre le support, le capot portant de préférence la zone de démarcation. Par exemple, le capot a une forme générale de disque percé en son centre, muni d'une jupe annulaire venant pincer la valve contre le support. Toutefois, le dispositif de distribution peut prendre bien d'autres formes. En particulier, le dispositif peut être un dispositif sans valve, pour la distribution de liquide sous forme de gouttes, notamment pour la distribution de collyre. Il peut également prendre la forme d'une pompe ou encore d'une valve.
- La zone de démarcation est réalisée par sérigraphie, tampographie (ou impression au tampon), marquage à chaud, impression par jet d'encre, ou encore par marquage laser.
- Le dispositif est configuré pour la distribution de liquide dans un oeil.

L'invention a par ailleurs pour objet l'utilisation d'un dispositif tel que décrit ci-dessus pour la distribution de liquide dans un oeil.

L'invention a également pour objet un procédé de fabrication d'un dispositif tel que décrit ci-dessus, comprenant une étape de marquage afin de réaliser la zone de démarcation, par exemple une étape de sérigraphie, de tampographie, de marquage à chaud, d'impression par jet d'encre, ou encore de marquage laser.

L'invention sera mieux comprise à la lecture de la description qui va suivre, donnée uniquement à titre d'exemple et faite en se référant aux dessins dans lesquels :
- la figure 1 est une vue en perspective d'un dispositif selon un mode de réalisation,
- la figure 2 est une vue similaire à la figure 1, selon une variante de réalisation,
- la figure 3 est une vue en coupe longitudinale du dispositif de la figure 1, muni d'un capuchon de protection,
- la figure 4 est une vue perspective d'un dispositif selon un deuxième mode de réalisation,
- les figures 5 à 9 sont des vues en perspectives de variantes du dispositif de la figure 4.

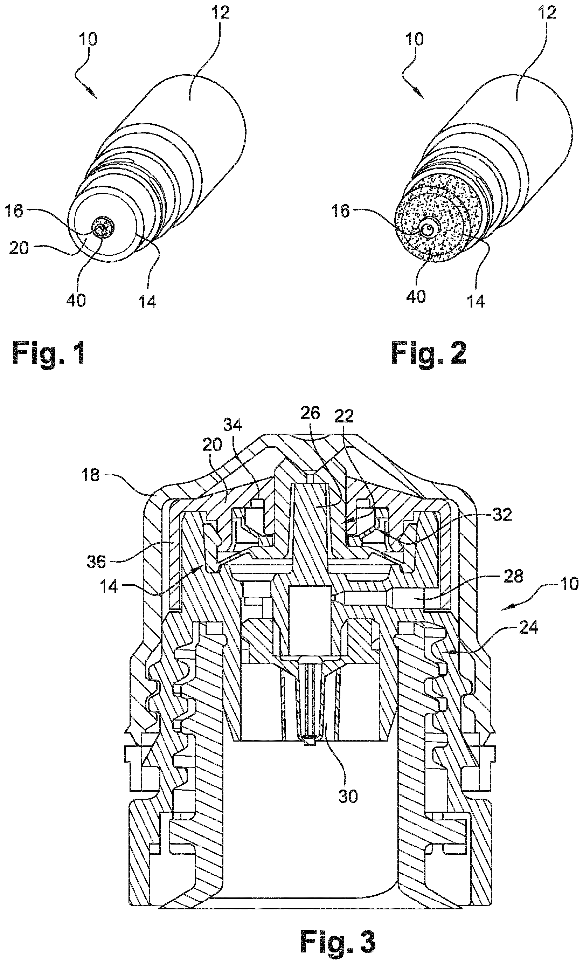

On a représenté sur la figure 1 un dispositif 10 de distribution de liquide sous forme de gouttes comprenant un réservoir 12 sur lequel est rapporté un embout de distribution 14, qui peut être surmonté d'un capuchon de protection 18, visible sur la figure 3.

L'embout 14 présente une extrémité supérieure de distribution 20 sur laquelle est ménagé un orifice 16 de distribution des gouttes délimité, dans cet exemple, par des moyens 16 de formation de gouttes. Plus précisément, toujours dans cet exemple, l'embout 14 est de forme générale sensiblement cylindrique, l'extrémité de distribution 20 ayant une forme générale de disque au centre duquel se trouve l'orifice de distribution 16. Cette extrémité 20 de forme de disque est de couleur non transparente, elle est par exemple blanche ou d'une autre couleur unie, si bien qu'elle représente une gêne pour l'utilisateur qui la rapproche très près de son oeil. Comme on peut le voir sur la figure 3, l'embout 14 comprend une valve 22 de distribution de gouttes, réalisée en matériau élastomère de façon à prendre, sous la pression du liquide (lorsque l'utilisateur appuie sur le réservoir 12), une configuration de passage du liquide. Les moyens 16 de formation de gouttes sont ménagés à l'extrémité de la valve 22, directement dans la matière élastomère. Ces moyens 16 sont de forme évasée conique. L'embout 14 comprend par ailleurs un support de valve 24, rapporté sur le réservoir 12, portant un pion 26 formant un siège contre lequel la valve 22 est plaquée et comportant également un conduit 28 de passage d'air, obturé par un organe 30 perméable à l'air empêchant l'introduction de bactéries dans le réservoir. L'embout 14 comporte également un élément de rappel 32, permettant de rappeler la valve 22 en configuration de blocage du liquide. Enfin, l'embout 14 comprend un capot 34 de plaquage de la valve contre le support 24, le capot comprenant l'extrémité 20, sous forme d'un disque sensiblement plat percé en son centre, ce disque étant muni d'une jupe annulaire extérieure 36, fixée sur le support 24, et d'une jupe intérieure de guidage de la valve 22.

Comme on peut le voir sur la figure 1, le dispositif comprend des moyens 40 de localisation visuelle de l'orifice, confondus ici avec les moyens 16 de formation de gouttes, par réalisation d'une zone de démarcation 40 ayant une couleur très contrastée par rapport à d'autres parties de l'extrémité de distribution. Dans cet exemple, la zone de démarcation 40 est réalisée à l'intérieur de l'orifice de distribution 16, par coloration des moyens de formation de gouttes. La coloration est très contrastée par rapport au reste de l'embout. Selon un exemple non limitatif, les moyens de formation de gouttes sont de couleur apparente foncée, éventuellement bleue, alors que le reste de l'embout est de couleur claire, blanche.

On notera que l'orifice de distribution 16 est réalisé sensiblement dans le même plan que la surface 20, si bien qu'il peut être difficile de le distinguer par rapport au reste de la surface 20 lorsque le dispositif 10 est disposé près de l'œil.Aussi, il est particulièrement utile de prévoir une zone de démarcation 40. On relève en particulier que la surface 20 est relativement étendue par rapport à la surface de l'orifice 16 de distribution de gouttes : la surface 20 a une aire supérieure à l'aire de l'orifice 16, en l'occurrence plus de deux, voire trois, fois supérieure.

Dans l'exemple de la figure 2, la zone de démarcation comprend la surface supérieure 20 de l'embout, du fait que le capot 34 est de couleur foncée, bleue, et que les moyens 16 de formation de gouttes sont de couleur claire, blanche ou transparente.

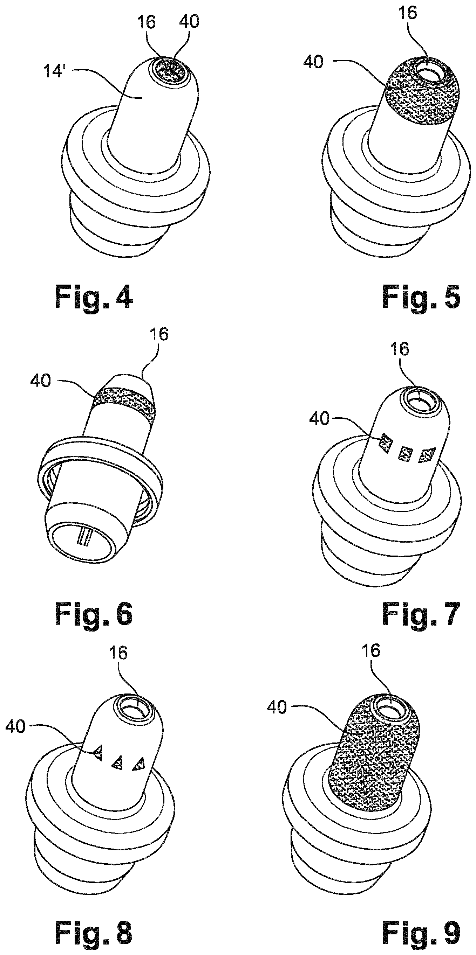

Dans le mode de réalisation de la figure 4, l'embout 14' est différent de l'embout 14 en ce qu'il se présente sous forme d'une buse de distribution formant nettement saillie du reste de l'embout. Aussi, dans ce mode de réalisation, la surface supérieure du dispositif est le sommet de la buse et est quasiment constituée par l'orifice de distribution de gouttes. La zone de démarcation 40 est réalisée ici par coloration, en foncé, de moyens de formation de gouttes 16. Dans cet exemple, toute la partie de formation de gouttes est colorée, toutefois on pourrait prévoir de la colorer partiellement.

Plusieurs variantes de zones de démarcation 40 sur le dispositif de la figure 4 peuvent être envisagées, de façon non limitative. Sur la figure 5, la zone de démarcation 40 est disposée autour de l'orifice de distribution 16, sous forme d'un hémisphère de couleur foncée, l'orifice de distribution 16 étant quant à lui réalisé dans une couleur claire. Sur la figure 6, la zone de démarcation 40 est également disposée autour de l'orifice de distribution 16, sous forme d'un anneau coloré continu de couleur foncée, l'orifice de distribution 16 étant réalisé dans une couleur claire. Cet anneau coloré peut être ménagé sur une partie intermédiaire de la buse, ou bien au voisinage immédiat de l'orifice de distribution, de sorte que l'anneau coloré soit disposé sur la buse juste à la périphérie de moyens de formation de gouttes. Sur la figure 7, la zone de démarcation 40 est similaire à celle de la figure 6, hormis le fait que l'anneau coloré est discontinu, par alignement circulaire de carrés ou de rectangles, toujours de couleur foncée. Sur la figure 8, de même que sur la figure 7, l'anneau 40 est discontinu, formé par un alignement circulaire de triangles. Enfin, sur la figure 9, la zone de démarcation 40 est composée par toute la buse de distribution, qui est réalisée de couleur foncée, alors que seul l'orifice de distribution 16 est réalisé de couleur claire.

On notera que, selon les modes de réalisation, la zone de démarcation de couleur foncée peut être réalisée par un matériau teinté dans la masse. De façon alternative, elle est réalisée par sérigraphie, marquage à chaud, impression par jet d'encre, ou encore par marquage laser, de façon à apposer une couleur de préférence foncée sur l'extrémité de distribution. Ainsi selon un mode de réalisation, le procédé de fabrication du dispositif 10 comprend une étape de marquage afin de réaliser la zone de démarcation, par exemple une étape de sérigraphie, de tampographie, de marquage à chaud, d'impression par jet d'encre, ou encore de marquage laser.

Le dispositif 10 décrit ci-dessus est tout particulièrement avantageux lorsqu'il est configuré ou utilisé pour la distribution de liquide dans un oeil.

L'invention n'est pas limitée aux exemples décrits ci-dessus. On comprend en particulier que toute façon de réaliser une démarcation de l'orifice 16 ou de son voisinage, par contraste visuel, est envisageable, de façon à aider l'utilisateur à localiser l'orifice, tout particulièrement lorsque l'utilisateur voit flou.

On comprend par ailleurs que les caractéristiques décrites peuvent être appliquées à d'autres dispositifs de distribution que ceux décrits en exemple, notamment à un dispositif de distribution sans valve, à une pompe, ou encore à une valve.

## Revendications

1. Dispositif (10) de distribution de liquide sous forme de gouttes comprenant un orifice (16) de distribution des gouttes ménagé sur une extrémité de distribution, **caractérisé en ce que** le dispositif comprend des moyens (40) de localisation visuelle de l'orifice, par réalisation d'une zone de démarcation (40) ayant une couleur très contrastée par rapport à d'autres parties de l'extrémité de distribution, le dispositif comprenant un embout de distribution (14) de forme générale sensiblement cylindrique, l'extrémité de distribution (20) ayant une forme générale de disque au centre duquel se trouve l'orifice de distribution (16), l'extrémité du dispositif se présentant sous forme d'une surface relativement étendue par rapport à la surface de l'orifice de distribution, l'aire de la surface de l'extrémité de distribution étant au moins deux fois supérieure à la surface de l'orifice de distribution, l'embout de distribution (14) ayant une dimension relativement réduite dans la direction longitudinale du dispositif, le disque étant de couleur opaque.

2. Dispositif selon la revendication précédente, dans lequel la zone de démarcation (40) est réalisée à l'intérieur de l'orifice de distribution (16).

3. Dispositif selon l'une quelconque des revendications précédentes, comprenant des moyens (16) de formation de gouttes, la zone de démarcation (40) étant obtenue par coloration de ces moyens de formation de gouttes, de préférence dans une couleur foncée.

4. Dispositif selon la revendication précédente, dans lequel les moyens de formation de gouttes sont composés par une forme évasée conique.

5. Dispositif selon l'une quelconque des revendications 1 à 2, comprenant des moyens (16) de formation de gouttes, la zone de démarcation étant disposée autour des moyens (16) de formation de gouttes, par exemple par une coloration foncée de tout ou partie d'un capot entourant les moyens (16) de formation de goutte.

6. Dispositif selon l'une quelconque des revendications précédentes, dans lequel le disque est de couleur blanche.

7. Dispositif selon l'une quelconque des revendications 1 à 6, le dispositif étant un dispositif sans valve.

8. Dispositif selon l'une quelconque des revendications 1 à 6, dans lequel l'embout comprend une valve (22) de distribution de gouttes, un support de valve (24) rapporté sur un réservoir (12) et un capot (34) de plaquage de la valve contre le support.

9. Dispositif selon la revendication précédente, dans lequel le capot a une forme générale de disque percé en son centre, muni d'une jupe annulaire venant pincer la valve contre le support.

10. Dispositif selon l'une quelconque des revendications précédentes, dans lequel la zone de démarcation (40) est réalisée par sérigraphie, tampographie, marquage à chaud, impression par jet d'encre, ou encore par marquage laser.

11. Dispositif selon l'une quelconque des revendications précédentes, le dispositif étant configuré pour la distribution de liquide dans un oeil.

12. Dispositif selon l'une quelconque des revendications précédentes, l'aire de la surface de l'extrémité de distribution étant au moins trois fois supérieure à la surface de l'orifice de distribution.

13. Procédé de fabrication d'un dispositif selon l'une quelconque des revendications précédentes, comprenant une étape de marquage afin de réaliser la zone de démarcation, par exemple une étape de sérigraphie, de tampographie, de marquage à chaud, d'impression par jet d'encre, ou encore de marquage laser.

## Patentansprüche

1. Vorrichtung (10) zur Ausgabe von Flüssigkeit in Tropfenform, umfassend eine Öffnung (16) zur Ausgabe der Tropfen, die an einem Ausgabeende vorgesehen ist, **dadurch gekennzeichnet, dass** die Vorrichtung Mittel (40) zur visuellen Lokalisierung der Öffnung umfasst, durch Ausbildung einer Abgrenzungszone (40), die eine Farbe hat, die gegenüber anderen Teilen des Ausgabeendes sehr kontrastreich ist, wobei die Vorrichtung einen Ausgabestutzen (14) allgemeiner, im Wesentlichen zylindrischer Form umfasst, wobei das Ausgabeende (20) eine allgemeine Scheibenform hat, in deren Mitte sich die Ausgabeöffnung (16) befindet, wobei das Ende der Vorrichtung die Form einer Oberfläche aufweist, die gegenüber der Oberfläche der Ausgabeöffnung eine relativ erweiterte Oberfläche aufweist, wobei die Fläche der Oberfläche des Ausgabeendes wenigstens zwei Mal so groß ist wie die Oberfläche der Ausgabeöffnung, wobei der Ausgabestutzen (14) eine Abmessung hat, die in der Längsrichtung der Vorrichtung relativ reduziert ist, wobei die Scheibe eine opake Farbe hat.

2. Vorrichtung nach dem vorhergehenden Anspruch, wobei die Abgrenzungszone (40) im Inneren der Ausgabeöffnung (16) ausgebildet ist.

3. Vorrichtung nach einem der vorhergehenden Ansprüche, umfassend Mittel (16) zur Bildung von Tropfen, wobei die Abgrenzungszone (40) durch Färbung dieser Mittel zur Bildung von Tropfen erhalten wird, bevorzugt in einer dunklen Farbe.

4. Vorrichtung nach dem vorhergehenden Anspruch, wobei die Mittel zur Bildung von Tropfen durch eine konische aufgeweitete Form gebildet sind.

5. Vorrichtung nach einem der Ansprüche 1 bis 2, umfassend Mittel (16) zur Bildung von Tropfen, wobei die Abgrenzungszone um die Mittel (16) zur Bildung von Tropfen herum angeordnet ist, zum Beispiel durch eine vollständige oder teilweise dunkle Färbung einer Kappe, welche die Mittel (16) zur Bildung von Tropfen umgibt.

6. Vorrichtung nach einem der vorhergehenden Ansprüche, wobei die Scheibe eine weiße Farbe hat.

7. Vorrichtung nach einem der Ansprüche 1 bis 6, wobei die Vorrichtung eine ventillose Vorrichtung ist.

8. Vorrichtung nach einem der Ansprüche 1 bis 6, wobei der Stutzen ein Ventil (22) zur Ausgabe von Tropfen, eine Ventilhalterung (24), die an einem Behälter (12) angebracht ist, und eine Kappe (34) zum Anpressen des Ventils gegen die Halterung umfasst.

9. Vorrichtung nach dem vorhergehenden Anspruch, wobei die Kappe eine allgemeine Scheibenform hat, die in ihrer Mitte durchbohrt ist und mit einer ringförmigen Schürze versehen ist, die das Ventil gegen die Halterung klemmt.

10. Vorrichtung nach einem der vorhergehenden Ansprüche, wobei die Abgrenzungszone (40) durch Siebdruck, Tampondruck, Heißprägung, Tintenstrahldruck oder auch durch Lasermarkierung realisiert ist.

11. Vorrichtung nach einem der vorhergehenden Ansprüche, wobei die Vorrichtung zur Ausgabe von Flüssigkeit als Auge ausgelegt ist.

12. Vorrichtung nach einem der vorhergehenden Ansprüche, wobei die Fläche der Oberfläche des Ausgabeendes wenigstens drei Mal so groß ist wie die Oberfläche der Ausgabeöffnung.

13. Verfahren zum Herstellen einer Vorrichtung nach einem der vorhergehenden Ansprüche, umfassend einen Schritt des Markierens, um die Abgrenzungszone zu realisieren, zum Beispiel einen Schritt des Siebdrucks, des Tampondrucks, der Heißprägung, des Tintenstrahldrucks oder auch der Lasermarkierung.

## Claims

1. Device (10) for dispensing liquid in the form of drops comprising a drop dispenser orifice (16) formed on a dispenser end, **characterized in that** the device comprises locator means (40) for visually locating the orifice, by forming a demarcation zone (40) having a colour that is highly contrasted relative to the other portions of the dispenser end, the device comprising a dispenser end piece (14) having a substantially cylindrical general shape, the dispenser end (20) having a general shape of a disc, the dispenser orifice (16) being located at the center of the disc, the end of the device being in the form of a surface that is relatively large compared to the surface of the dispenser orifice, the surface of the dispenser end having an area that is at least two times greater than the area of the dispenser orifice surface, the dispenser end piece (14) having a relatively short dimension in the longitudinal direction of the device, the disc being of opaque colour.

2. Device according to the preceding claim, in which the demarcation zone (40) is formed inside the dispenser orifice (16).

3. Device according to any one of the preceding claims, comprising drop-forming means (16), the demarcation zone (40) being obtained by coloring the drop-forming means, preferably in a dark colour.

4. Device according to the preceding claim, in which the drop-forming means are of a conical flared shape.

5. Device according to any one of claims 1 to 2, comprising drop-forming means (16), the demarcation zone being arranged around the drop-forming means (16), for example by dark coloring of all or part of a cap surrounding the drop-forming means (16).

6. Device according to any one of the preceding claims, in which the disc is white in colour.

7. Device according to any one of claims 1 to 6, the device being a device without any valve.

8. Device according to any one of claims 1 to 6, in which the dispenser end piece comprises a valve (22) for dispensing drops, a valve support (24) fitted on a reservoir (12) and a cap (34) for pressing the valve against the support.

9. Device according to the preceding claim, in which the cap has a general shape of a disc perforated at its center, and provided with an annular skirt pinching the valve against the support.

10. Device according to any one of the preceding claims, in which the demarcation zone (40) is realised by silk-screen printing, pad printing, hot marking, printing by ink jet, or by laser marking.

11. Device according to any one of the preceding claims, the device being configured for dispensing liquid into an eye.

12. Device according to any one of the preceding claims, the area of the surface of the dispenser end is at least three times greater than the area of the dispenser orifice surface.

13. Method of manufacturing a device according to any one of the preceding claims, comprising a marking step in order to realise the demarcation zone, for example a step of silk-screen printing, pad printing, hot marking, printing by ink jet, or laser marking.
